# EUROPÄISCHE PATENTANMELDUNG

(11) **EP 2 634 246 A1**
(43) Veröffentlichungstag der Anmeldung: **04.09.2013**
(21) Anmeldenummer: 12157894.2
(22) Anmeldetag: 02.03.2012
(51) Int. Cl.: C12M 1/33, C12M 1/34, C12M 3/08

(54) **Vorrichtung und Verfahren zur Identifikation, Separation und/oder zelltypspezifischen Manipulation wenigstens einer Zelle eines Zellsystems sowie von Mikroorganismen**

(71) Anmelder: Justus-Liebig-Universität Giessen, 35390 Giessen (DE)
(72) Erfinder: Walther, Thomas, 16278 Angermünde (DE); Postema, Michiel, 2661 SG Bergschenhoek (NL)
(74) Vertreter: Stumpf, Peter

(57) **Zusammenfassung**

Die vorliegende Erfindung betrifft eine Vorrichtung zur Identifikation, Separation und/oder zelltypspezifischen Manipulation wenigstens einer Zelle eines Zellsystems sowie von Mikroorganismen, ein Verfahren zur Identifikation, Separation und/oder zelltypspezifischen Manipulation wenigstens einer Zelle eines Zellsystems sowie von Mikroorganismen und die Verwendung der Vorrichtung zur Durchführung des erfindungsgemäßen Verfahrens. Dabei wird das Zellsystem mit zelltypspezifischen Frequenzen beschallt, die mit einer Schallgebungseinheit erzeugbar sind.

## Beschreibung

Die Erfindung betrifft eine Vorrichtung zur Identifikation, Separation und/oder zelltypspezifischen Manipulation wenigstens einer Zelle eines Zellsystems sowie von Mikroorganismen gemäß dem Oberbegriff von Anspruch 1, ein Verfahren zur Identifikation, Separation und/oder zelltypspezifischen Manipulation wenigstens einer Zelle eines Zellsystems sowie von Mikroorganismen gemäß Anspruch 7 und die Verwendung einer solchen Vorrichtung gemäß Anspruch 9.

### DEFINITIONEN:

Mikroorganismus: Kleinstlebewesen, mikroskopisch kleine Einzeller oder Mehrzeller, beispielsweise Bakterien, Protozoen, Pilze, Hefen und Algen.

### Zellsystem:

a) Monozellsystem: Jedes organische System, das nur einen Zelltyp enthält, beispielsweise hyperreine Zellsysteme oder
b) Multizellsystem: Jedes organische System, das mehr als einen Zelltyp enthält, beispielsweise Zellkulturen oder Organismen.

Primäre Zellkultur: Nicht immortalisierte (d.h. sterbliche) Zellkultur, welche direkt aus einem Gewebe gewonnen wurde.

Schallgebungseinheit: Bauteil, welches aus elektrischer Spannung akustische Signale in Form von Schallwechseldrücken, auch im Ultraschallbereich, erzeugt.

Sonopulation: Veränderung oder Manipulation von Zellen durch Schall (akustische Wellen).

Zelltyp: Zellen eines Zelltyps üben dieselbe Funktion in einem Organismus aus. Daher ähneln sie sich auch meist in ihrem Aussehen, ihrer Struktur und ihrem inneren Aufbau. Aber auch einzelne Einzeller können als ein spezieller Zelltyp aufgefasst werden.

Zellkulturen spielen in vielen Bereichen der Life Sciences eine große Rolle, insbesondere in der biotechnologischen und biomedizinischen Forschung. Aber auch in der Diagnostik und Therapie einer Vielzahl von Erkrankungen, sowohl bei Menschen als auch bei Tieren, werden mittlerweile Zellkulturen eingesetzt. Aufgrund zunehmender Vorbehalte gegenüber Tierversuchen wird die Etablierung biologisch relevanter In-vitro-Testsysteme, und damit Zellkulturen, immer bedeutender. Primärzellen sind die Grundlage zur Entwicklung komplexer In-vitro-Modelle und Testmethoden (Assays) und bieten somit eine hervorragende Alternative zu Tierversuchen. Die Herstellung von Primärkulturen aus einer Gewebeprobe sowie deren weitere Kultivierung für experimentelle Zwecke stellt eine große Herausforderung dar.

Dazu ist die zelltypspezifische Trennung der Zellen von entscheidender Bedeutung, um saubere Primärzellkulturen zu erhalten. Bisher werden die Zellen mittels Durchflusszytometrie getrennt. Von Nachteil dabei ist jedoch, dass die Zellen spezifisch angefärbt werden müssen, beispielsweise mit Fluoreszenzfarbstoffen, was nicht immer möglich ist oder wodurch die zu separierenden Zellen nicht voneinander unterschieden werden können. Eine weitere Möglichkeit besteht in der Markierung der Zellen mit spezifischen und markierten Antikörpern. Dabei besteht jedoch das Problem darin, dass für viele Zelltypen keine spezifischen Antikörper zur Verfügung stehen und diese erst noch aufwendig entwickelt werden müssen. Dieses Verfahren ist zudem sehr zeitaufwendig und kostenintensiv. Weiterhin kann dieses Verfahren nicht für Zellen, die sich nicht frei flottierend in einer Zellkultur befinden, angewendet werden. Das trifft vor allem auf Zellen in einem Multizellsystem, wie sie ein Organismus dargestellt, zu. Unterschiedliche Zelltypen können weiterhin durch morphologische Untersuchungen sortiert werden. Dieses Verfahren ist jedoch sehr stark vom Geschick und von der Erfahrung der Person abhängig, die diese Trennung durchführt. Weiterhin ist diese Methode extrem zeitaufwendig. Für die Untersuchung selbst müssen die Zellen dem Multizellsystem entnommen werden, wodurch die Sterilität der Zellen beeinträchtigt wird. In Organismen ist dies oftmals nicht durchführbar, ohne dabei den Organismus zu schädigen.

Es ist bekannt, dass Teilchen oder Partikel in einer Flüssigkeit, z.B. verkapselte Mikrogasbläschen, bei der Beschallung mit Ultraschallwellen eine andere Resonanzfrequenz aufweisen als freie Gasbläschen in dieser Flüssigkeit und sich so von diesen unterscheiden lassen *(*Postema M. Fundamentals of Medical Ultrasonics. Spon Press, London 2011*).* Basierend auf ihren akustischen Eigenschaften sind Mikrogasbläschen auch als Ultraschaflkontrastmittel geeignet und finden dort Anwendung, z.B. in der bildgebenden Diagnostik. Dabei ziehen sich Teilchen mit gleichen akustischen Eigenschaften an, während sich Teilchen mit unterschiedlichen akustischen Eigenschaften abstoßen. Bei der gegenseitigen Anziehung von Teilchen mit gleichen akustischen Eigenschaften kann es auch zur Fusion mehrerer Mikrogasbläschen kommen. Dieses Phänomen kann durch die sekundären Bjerknes-Kräfte erklärt werden.

Die Veröffentlichung Kotopoulis S., Postema M. Microfoam formation in a capillary. Ultrasonics 2010;50:206-268 beschreibt die Bildung eines Mikroschaums durch die Manipulation von Mikrogasbläschen mit Ultraschall. Durch die Anregung von Mikrogasbläschen mit Ultraschall beginnen sich die einzelnen Mikrogasbläschen anzuziehen und bilden auf diese Weise ein Cluster bestehend aus Mikrogasbläschen. Dies geschieht unverzüglich, schon in der ersten Sekunde der Einwirkung des Ultraschalls. Werden die so entstandenen Mikrogasbläschen-Cluster weiterhin dem Ultraschall ausgesetzt, so beginnen sich die entstandenen Cluster zu größeren Clustern zusammenzulagern und bilden auf diese Weise einen Mikroschaum aus. Solange sich die zum Mikroschaum zusammengelagerten Cluster im Ultraschallwellenfeld befinden, verhalten sich die Cluster als eine Einheit.

Weiterhin beschreibt die Veröffentlichung Jönsson H., Holm C., Nilsson A., Petersson F., Johnsson P., Laurell T. Ultrasound can radically reduce embolic load to brain after cardiac surgery. Ann. Thorac. Surg. 2004;78:1572-1578 die Trennung von Partikeln in Flüssigkeiten mittels akustischer stehender Wellen. In einem speziellen Beispiel wird gezeigt, wie Lipidteilchen mittels Ultraschall von Blutproben und den darin vorliegenden anderen Kompartimenten (Plasma, Blutzellen, Zucker usw.) separiert und abgetrennt werden können. Dadurch können insbesondere Mikroembolien, die durch einen erhöhten Lipidgehalt im Blut entstehen und insbesondere nach Herzoperationen (Bypass-Operationen) vorkommen, verhindert werden.

Jeder Zelltyp und auch jeder Mikroorganismus sollte ein spezifisches akustisches Verhalten aufweisen, welches abhängig ist von seiner Kompressibilität, Dichte und Geometrie. Dadurch weist jeder Zelltyp oder Mikroorganismus eine charakteristische Schallfrequenz auf, an der die Zelle oder der Mikroorganismus am stärksten reagiert und somit von den Schallwellen beeinflusst wird. Diese charakteristische Schallfrequenz wird als seine spezifische Resonanzfrequenz bezeichnet. Wird eine Zelle einer Schallwelle dieser Resonanzfrequenz ausgesetzt, antwortet die Zelle mit einem dynamischen Signal in Form einer Oszillation. Wird jedoch eine bestimmte Amplitude dieser Resonanzfrequenz überschritten, so kann die Zelle oder der Mikroorganismus so stark in Schwingung geraten, dass diese oder dieser dabei zerstört wird. Die Veröffentlichung Delalande A., Kotopoulis S., Rovers T., Pichon C., Postema M. Sonoporation at low mechanical index. Bubble Science, Engineering and Technology 2011;3:3-11 zeigt die akustische Aktivität von bestimmten Krebszellen.

WO 01/00084 A1 offenbart eine Vorrichtung und ein Verfahren zur nichtinvasiven Zerstörung von (Tumor)Gewebe durch Behandlung dieses Gewebes mit von Schall erzeugten Stoßwellen vorbestimmter Frequenzen. Diese Stoßwellen bringen nur das entartete Gewebe ernsthaft zum Schwingen, wodurch das umliegende Gewebe kaum beeinflusst wird. Die Amplitude der entsprechenden Stoßwelle ist so hoch, dass die mechanische Reibung im Gewebe für die Zerstörung von Zellen, durch Kavitation und Heizung, hoch genug ist. Nachteil dabei ist jedoch, dass alle angestrahlten Zellen dabei zerstört werden. Weiterhin dringen Stoßwellen tief in das Gewebe ein, wodurch auch Schädigungen durch diese Stoßwellen tief im Gewebe erzeugt werden können.

US 6,406,429 B1 offenbart eine Vorrichtung und eine Methode zur nichtinvasiven Detektion von zystischen Strukturen und Vorstufen von Krebszellen in jeglichen Geweben mittels Ultraschallwellen. Dabei handelt es sich um ein bildgebendes Verfahren. Maßnahmen zur Beseitigung einzelner Zelltypen sind nicht offenbart.

In der Veröffentlichung Kotopoulis S., Schommartz A., Postema M. Sonic cracking of blue-green algae. Applied Acoustics 2009;70:1306-1312 wird beschrieben, wie Ultraschallwellen der Frequenz, wie sie im klinisch-diagnostischen Bereich eingesetzt wird (200 kHz bis 2.2 MHz), dazu genutzt werden kann, die in Blau-Grün-Algen (Cyanobakterien) vorhandenen Heterozysten, welche die Auftrieb gebenden Zellen in den Algen sind, zu zerstören. Dadurch sinken die Algen zu Boden. Die zu Boden gesunkenen Algen weisen weiterhin vollkommen intakte und Chlorophyll enthaltende Chloroplasten auf, die durch die Ultraschalleinwirkung unberührt bleiben. Es ist demnach möglich, spezifische Zellen mit Ultraschall zu manipulieren oder zu zerstören, währenddessen andere Zelltypen in der Umgebung nicht davon beeinflusst werden.

Aufgabe der Erfindung ist es daher, eine Vorrichtung und ein Verfahren zur Identifikation, Separation und/oder zelltypspezifischen Manipulation (Sonopulation), wobei Manipulation auch das gezielte Abtöten von Zellen umfassen kann, wenigstens einer Zelle eines Zellsystems sowie von Mikroorganismen zur Verfügung zu stellen, ohne dabei die Sterilität der Zellen zu beeinträchtigen oder dabei die Zellen aus dem Zellsystem entnehmen zu müssen.

Hauptmerkmale der Erfindung sind im kennzeichnenden Teil der Ansprüche 1, 7 und 9 angegeben. Ausgestaltungen sind Gegenstand der Ansprüche 2 bis 6, 8 und 10.

Die Erfindung sieht eine Vorrichtung zur Identifikation, Separation und/oder zelltypspezifischen Manipulation wenigstens einer Zelle eines Zellsystems sowie von Mikroorganismen vor, wobei die Vorrichtung eine Schallgebungseinheit, eine Kontrolleinheit und eine Aufnahmeeinheit aufweist, wobei die Schallgebungseinheit ein piezo-elektrisches Bauteil aufweist, welches mit einer Frequenz von mehr als 5 MHz gesteuert wird und eine schmale Bandbreite hat.

Mit der erfindungsgemäßen Vorrichtung wird die Voraussetzung für eine schnelle und bequeme Separierung und Identifizierung von unterschiedlichen Zelltypen in einem Multizellsystems ermöglicht, ohne dabei die einzelnen Zellen zu beschädigen oder die sterilen Bedingungen der Zellkultur zu beschädigen. Eine Entnahme der einzelnen Zellen aus dem Multizellsystem ist nicht zwingend erforderlich. Dies ist insbesondere für primäre Zellkulturen sehr wichtig. Auch die Manipulation (Sonopulation), wie z.B. Transfektion oder Transformation, einzelner Zelltypen in einem Zellsystem oder einem Multizellsystem kann auf diese Weise durchgeführt werden. Mit der Kontrolleinheit kann dabei gegebenenfalls direkt der Erfolg dieser Manipulation beobachtet werden. Weiterhin bietet diese Vorrichtung den Vorteil, die Resonanzfrequenz eines speziellen Zelltyps zu ermitteln und diesen auf diese Weise mit ebendieser Resonanzfrequenz gezielt zu beeinflussen. Insbesondere bei Zellen, die mit Viren oder mit sonstigen Parasiten befallen sind, stellt dies eine Möglichkeit dar, diese aus der Zellkultur zu separieren und anschließend zu entfernen oder gegebenenfalls in der Zellkultur zu zerstören, ohne dabei andere Zellen zu beeinflussen oder zu schädigen.

In einer bevorzugten Ausführungsform der erfindungsgemäßen Vorrichtung, ist das piezo-elektrische Bauteil ein piezo-elektrischer Kristall, eine Piezo-Keramik oder ein Piezo-Polymer. Die ältesten und bekanntesten piezo-elektrischen Bauteile zur Ultraschallerzeugung stellen die piezo-elektrischen Kristalle dar. Darunter fallen beispielsweise die stabile α-Quarz-Modifikation, Lithiumniobat oder Galliumorthophosphat. Weitere piezo-elektrische Kristalle sind Berlinit, Turmaline und Seignettensalz. Zu den piezo-elektrischen Keramiken gehören beispielsweise Bleititanat (PT), Bleizirkonattitanat (PZT), Bismuttitanat, Bariumtitanat und Bleimetaniobat (PMN). Das gängigste Piezo-Polymer ist beispielsweise Polyvinylidenfluorid (PVDF) oder ein Copolymer von Polyvinylidenfluorid und Trifluorethylen. Somit kann das piezo-elektrische Bauteil optimal an die erforderliche Bauform oder die Nutzung angepasst werden und dadurch optimal an die entsprechenden Anforderungen (Kopplungsfaktor, Querkopplung, Schallwellenwiderstand (akustische Impedanz und Bandbreite) angepasst und abgestimmt werden.

Weiterhin ist es vorteilhaft, wenn das piezo-elektrische Bauteil eine Resonanz von 7 MHz pro Millimeter aufweist, um so eine bestmögliche Umsetzung elektrischer Signale in mechanische Signale zu erhalten. Piezo-elektrische Bauteile mit einer spezifischen Resonanzfrequenz weisen bei Anregung mit einer Spannung bei dieser Resonanzfrequenz die höchste Amplitude des erzeugten Ultraschalls auf. Somit sollte die Resonanzfrequenz in dem Frequenzbereich liegen, in welchem die Ultraschallfrequenz benötigt wird. Eine große Streuung der Resonanzfrequenzen würde eine Qualitätsverschlechterung des ausgegebenen Ultraschallsignals (Schalldruck) bedeuten. Aber auch andere Resonanzen des piezo-elektrischen Bauteils sind denkbar.

In einer weiteren vorteilhaften Ausführungsform ist das piezo-elektrische Bauteil ein Wafer, der in einer Orientierung von 36° zur Y-Achse aus einem Lithiumniobatkristall (LiNbO₃) geschnitten ist. Lithiumniobat ist ein piezoelektrischer Kristall, der auch bei hoher angelegter Spannung nicht zerbricht und somit auch zur Erzeugung von Ultraschall in hohen Frequenzbereichen (250 kHz bis 40 MHz) geeignet ist, auch wenn dieses Material sehr dünn geschnitten wurde. Des Weiteren weist Lithiumniobat eine sehr hohe Resonanz auf. In einer bevorzugten Ausführungsform, ist der piezo-elektrische Wafer aus Lithiumniobat (LiNbO₃) gefertigt und weist eine Dicke von 0,5 mm und einen Durchmesser von 7,6 cm auf. Solche Wafer sind unter anderem über Boston Piezo-Optics. Inc., Boston, MA, USA, zu beziehen.

Zur Untersuchung oder Beobachtung der Separation und/oder zelltypspezifischen Manipulation wenigstens einer Zelle eines Zellsystems sowie von Mikroorganismen ist es ein großer Vorteil, wenn die Kontrolleinheit der erfindungsgemäßen Vorrichtung eine Vergrößerungsoptik, bevorzugt ein Binokular oder ein Inversmikroskop, ist. So kann während der Beschallung der Probe mit Ultraschall sofort das Ergebnis kontrolliert werden und die notwendige Beschallung auf ein Minimum reduziert werden, um so eine eventuelle Schädigung anderer Zellen komplett ausschließen zu können.

Es ist vorteilhaft, wenn die Aufnahmeeinheit eine Halterung für eine Zellkulturschale, Petrischale o.dgl. ist. Insbesondere dann können die zu identifizierenden, separierenden oder zu manipulierenden Zellen in dem Zellsystem in die Vorrichtung eingebracht werden, ohne dabei aus ihrem Umgebungsmedium entnommen werden zu müssen, wodurch die sterile Umgebung gewährleistet bleibt. Auch ein eventuell schädlicher Luftkontakt und insbesondere Sauerstoffkontakt kann ausgeschlossen werden, wenn die Zellen in Ihrem Nährmedium verbleiben können. Insbesondere bei Zellkulturen erweist sich dies als vorteilhaft, da üblicherweise Zellkulturen in Petrischalen angelegt sind. Aber auch andere Proben können leicht und bequem in Zellkulturschalen oder Petrischalen eingebracht werden und ideal gehandhabt werden. Auch das Einbringen in Zellkulturschränke oder Brutschränke ist so gut möglich.

Bei einem Verfahren zur Identifikation, Separation und/oder zelltypspezifischen Manipulation wenigstens einer Zelle eines Zellsystems sowie von Mikroorganismen mit einer erfindungsgemäßen Vorrichtung sieht die Erfindung vor, dass das Verfahren die Schritte
a) Einbringen des Zellsystems in die Aufnahmeeinheit der Vorrichtung und
b) Beschallen des Zellsystems mit der Schallgebungseinheit mit einer zelltypspezifischen Frequenz umfasst.

Von besonderem Vorteil ist es, wenn das Verfahren wenigstens einen der Schritte
■ Unterscheidung und Sortierung der Zellen des Multizellsystems oder des Zellsystems in primären Zellkulturen;
■ Erstellung eines Resonanzprofils für die beschallten Zellen des Multizellsystems oder des Zellsystems;
■ Transformation der beschallten Zellen des Multizellsystems oder des Zellsystems;
■ Transfektion der beschallten Zellen des Multizellsystems oder des Zellsystems;
■ Diskriminierung der Zellen des Multizellsystems oder des Zellsystems anhand ihres Genotyps, wobei die Zellen bevorzugt Spermien sind;
■ Isolation und Zerstörung von Zellen des Multizellsystems oder des Zellsystems, wobei die Zellen bevorzugt Virus- oder Parasiten-tragende Zellen oder Tumorzellen sind;
■ Einwirken auf die Zellen des Multizellsystems oder des Zellsystems zur Vermeidung von Restenosen, wobei die Zellen bevorzugt Zellen der Neointima sind;
■ Isolation bzw. Identifikation von verschiedenen Subpopulationen innerhalb des Multizellsystems oder des Zellsystems, wobei das Multizellsystem oder das Zellsystem bevorzugt Mikroorganismen in gemischten Populationen aufweist;
■ Aktivierung von spezifischen dendritischen Zellen;
■ Manipulation von intrazellulärem Signalling umfasst.

Das erfindungsgemäße Verfahren ist demnach dazu geeignet, Zellen eines Multizellsystems nach unterschiedlichen Zelltypen zu trennen. Dies ist insbesondere in Zellkulturen und besonders in primären Zellkulturen von besonderer Bedeutung. Die Zellen in der Zellkultur können schnell und präzise sortiert und getrennt werden. Weiterhin kann für die Zellen des Multizellsystems ein Resonanzprofil erstellt werden, womit nur diese spezifischen Zelltypen von der Ultraschallfrequenz beeinflusst oder manipuliert werden können.

Weiterhin wird mit dem Verfahren die gezielte Transformation oder Transfektion eines bestimmten Zelltyps in einem Multizellsystem mithilfe von Ultraschall ermöglicht, ohne zuvor eine manuelle Trennung der Zelltypen vornehmen zu müssen. Zellen, insbesondere Spermien, können anhand Ihres Genotyps diskriminiert werden. Ein weiterer Vorteil des erfindungsgemäßen Verfahrens liegt darin, dass spezielle Zellen, und dabei insbesondere mit Virus- oder Parasiten befallene Zellen oder sonstig entartete Zellen, insbesondere Tumorzellen, mit der Beschallung mit Ultraschall einer speziellen Resonanzfrequenz, diese Zellen in dem Multizellsystem von den anderen Zellen isoliert und mit Ultraschall zerstört werden können. Dabei bleiben die übrigen Zellen im Multizellsystem unbeeinflusst.

Auch zur Vermeidung von Restenosen, nach einer behandelten Stenose (Gefäßverengung), kann die Behandlung eines Multizellsystems mit Ultraschall eingesetzt werden. Dies ist insbesondere für Zellen der Neointima von Bedeutung. Auch die Isolierung oder Identifizierung von Subpopulationen verschiedener Zelltypen innerhalb eines Multizellsystems kann mit dem erfindungsgemäßen Verfahren durchgeführt werden. Somit bietet das Verfahren viele Vorteile und neue Möglichkeiten zur Identifikation, Separation und/oder zelltypspezifischen Manipulation von unterschiedlichen Zellen in einem Multiellsystem. Die Aktivierung von dendritischen Zellen, die als Zellen des Immunsystems zu dem Schlüsselzelltyp bei inflammatorischen Prozessen zählen, dient zur Erzeugung einer Immunantwort oder zur Mobilisierung der Immunabwehr. Die Manipulation von intrazellulären Signalprozessen erlaubt unter anderem die Stimulation der Proliferation von Stamm- oder Progenitorzellen bei spezifischen Erkrankungen, die regenerative Prozesse zur Wundheilung benötigen. Aber auch in der translationalen Medizin lässt die Beeinflussung von intrazellulären Signalprozessen mittels Ultraschall völlig neue Ansätze zu. Mit der Beeinflussung von intrazellulären Signalprozessen innerhalb der Zelle besteht auch die Möglichkeit, eine Apoptose einzuleiten und den gezielten Zelltod einzuleiten. Dies ist insbesondere in der Krebsforschung von enormer Bedeutung.

Mit der optimalen Einstellung der akustischen Parameter bezüglich eines Zelltyps und der anschließenden Beschallung der Zellen mit diesen Schallfrequenzen, kann die Permeabilität der Zellmembran erhöht werden, womit eine verbesserte Aufnahme von Medikamenten, Kontrastmitteln, Färbemitteln usw. einhergeht, ohne dadurch unerwünschte Nebeneffekte in beispielsweise intrazellulären Signalprozessen oder zellphysiologischen Abläufen zu erzeugen.

Des Weiteren sieht die Erfindung die Verwendung der erfindungsgemäßen Vorrichtung zur Durchführung des erfindungsgemäßen Verfahrens vor.

Die Verwendung des Verfahrens umfasst wenigstens einen der Schritte
■ Unterscheidung und Sortierung der Zellen des Multizellsystems oder des Zellsystems in primären Zellkulturen;
■ Erstellung eines Resonanzprofils für die beschallten Zellen des Multizellsystems oder des Zellsystems;
■ Transformation der beschallten Zellen des Multizellsystems oder des Zellsystems;
■ Transfektion der beschaflten Zellen des Multizellsystems oder des Zellsystems;
■ Diskriminierung der Zellen des Multizellsystems oder des Zellsystems anhand ihres Genotyps, wobei die Zellen bevorzugt Spermien sind;
■ Isolation und Zerstörung von Zellen des Multizellsystems oder des Zellsystems, wobei die Zellen bevorzugt Virus- oder Parasiten-tragende Zellen oder Tumorzellen sind;
■ Einwirken auf die Zellen des Multizellsystems oder des Zellsystems zur Vermeidung von Restenosen, wobei die Zellen bevorzugt Zellen der Neointima sind;
■ Isolation bzw. Identifikation von verschiedenen Subpopulationen innerhalb des Multizeffsystems oder des Zellsystems, wobei das Multizellsystem oder das Zellsystem bevorzugt Mikroorganismen in gemischten Populationen aufweist;
■ Aktivierung von spezifischen dendritischen Zellen;
■ Manipulation von intrazellulärem Signalling.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: einen Aufbau einer Vorrichtung zur Identifikation, Separation und/oder zelltypspezifischen Manipulation wenigstens einer Zelle eines Zellsystems sowie von Mikroorganismen;
- Fig. 2A: CHO-Zellen vor und nach einer Behandlung mit Ultraschall;
- Fig. 2B: HEK-Zellen vor und nach einer Behandlung mit Ultraschall.

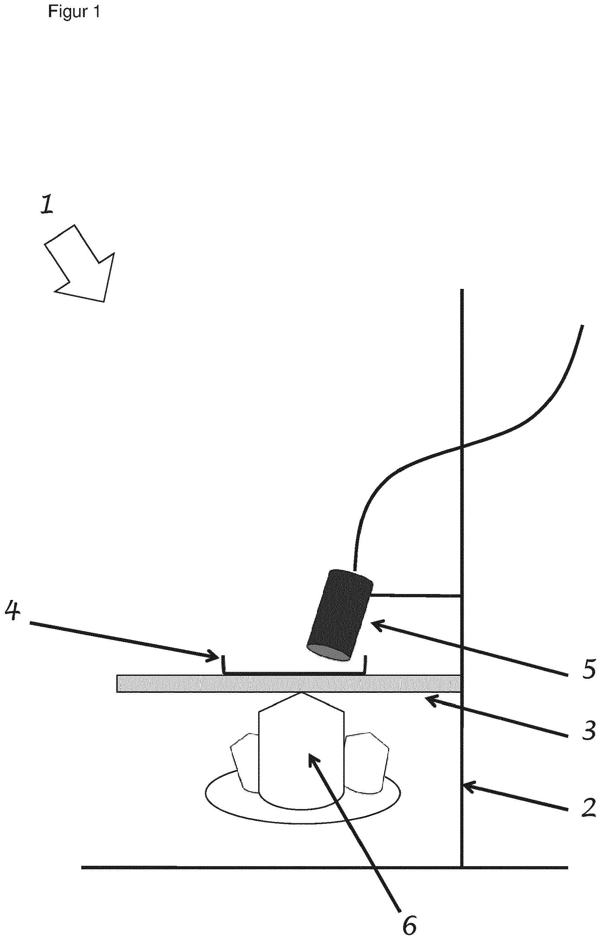

Die allgemein mit 1 bezeichnete erfindungsgemäße Vorrichtung (Fig. 1) zur Identifikation, Separation und/oder zelltypspezifischen Manipulation wenigstens einer Zelle eines Zellsystems sowie von Mikroorganismen weist eine Kontrolleinheit 2 auf, die aus einem biologischen InversMikroskop MBL 3200 der Firma A. Krüss Optronic GmbH, Hamburg, Germany, besteht und unterhalb einer Probe eine Vergrößerungsoptik 6 aufweist. An der Kontrolleinheit 2 ist eine Aufnahmeeinheit 3 für eine Petrischale 4 montiert. Die Petrischale 4 hat einen Durchmesser von 10 cm und ist mit vom Boden der Petrischale 4 abgelösten Zellen, eines Zellsystems, das sich in Dulbecco's Modified Eagle Medium (DMEM) - einem standardisierten Nährmedium für Zellkulturen - befindet, gefüllt. Dabei können die Zellen in der Petrischale 4 zum Beispiel Ovarialzellen des chinesischen Hamsters *(Cricetulus griseus)* (CHO-Zellen), menschliche embryonale Nierenzellen (HEK-Zellen), Endothelzellen der Aorta vom Rind (BAEC-Zellen) oder embryonale Mäusefibroblast-Zellen (3T3/NIH-Zellen) sein.

Der benötigte Ultraschall wird mit einer Schallgebungseinheit 5 erzeugt, wobei die Schallgebungseinheit 5 ein piezo-elektrisches Bauteil aufweist, welches mit einer Frequenz von mehr als 5 MHz gesteuert wird und eine schmale Bandbreite hat. Die Schallgebungseinheit 5 besteht dabei aus einem 7 MHz Transducer mit einem piezo-elektrischen Kristall als piezoelektrischem Bauteil, welches aber auch eine Piezo-Keramik oder ein Piezo-Polymer sein kann. Der piezo-elektrische Kristall besteht aus Lithiumniobat (LiNbO₃), welches mit einer Orientierung von 36° zur Y-Achse geschnitten ist und eine Resonanz von 7 MHz pro Millimeter aufweist. Diese Schallgebungseinheit 5 ist in einem Winkel von 17° über der Petrischale 4 angeordnet.

Ein nicht gezeigter Frequenzgeber AFG3102 (Tektronix, Everett, WA, USA) steuert die Schallgebungseinheit 5 mit einer Grundfrequenz einer kontinuierlichen Welle. Das Signal des nicht gezeigten Frequenzgebers wird vorher durch einen 20 dB Dämpfungsregler (nicht gezeigt) geleitet, bevor es als Eingangssignal in einen ebenfalls nicht gezeigten Kraftverstärker 2100L 50 dB RF (Electronics & Innovation Ltd., Rochester, NY, USA) geführt wird und von dort erst zur Schallgebungseinheit 5 kommt, wo der erzeugte Ultraschall in die Probe in der Petrischale 4 geleitet wird.

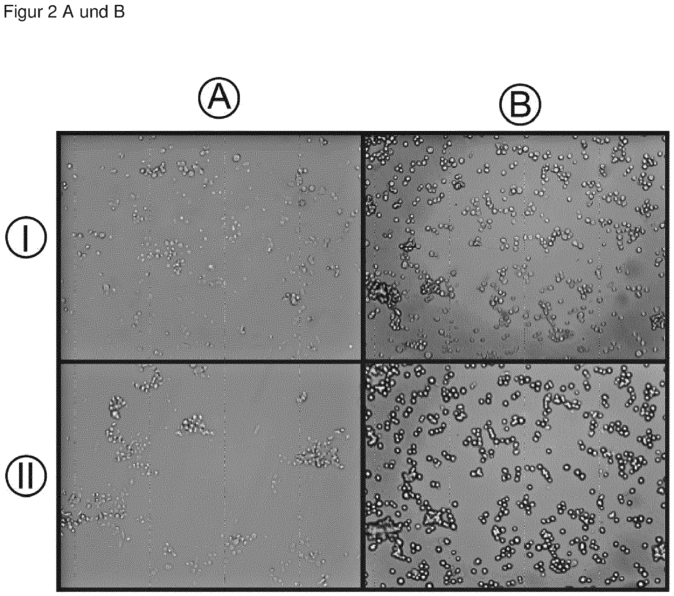

Fig. 2 A zeigt Eizellen des chinesischen Hamsters (CHO-Zellen) in einer Petrischale 4. Das obere Bild (2 A I) zeigt die Zellen vom Boden der Petrischale 4 abgelöst in einem Nährmedium vor der Ultraschallbehandlung. Das untere Bild (2 A II) zeigt die gleiche Probe nach einer Behandlung von 30 Sekunden mit Ultraschall einer Frequenz von 7 MHz. Die Zellen haben sich zu größeren und dicht gepackten Clustern zusammengelagert. Diese Clusterbildung setzt schon nach wenigen Sekunden der Ultraschallbehandlung ein.

Fig. 2 B zeigt menschliche embryonale Nierenzellen (HEK-Zellen) in einer Petrischale 4, die zuvor vom Boden der Petrischale 4 abgelöst wurden und frei im Nährmedium beweglich sind. Das obere Bild (2 B I) zeigt die Zellen in der Nährlösung vor der Ultraschallbehandlung. Das untere Bild (2 B II) zeigt die Zellen nach 30 Sekunden Ultraschallbehandlung mit einer Frequenz von 7 MHz. Diese Zellen zeigen, im Gegensatz zu den CHO-Zellen, keine Cluster-Bildung. Unterschiedliche Zelltypen zeigen demnach bei Ultraschallbehandlung mit derselben Frequenz unterschiedliches Verhalten. Jedes der gezeigten Bilder repräsentiert eine Probenfläche von 960x720 (μm)².

Eine Mischung beider Zelltypen (CHO-Zellen und HEK-Zellen) in einer Probenschale 4 zeigt ebenfalls eine Clusterbildung, wobei sich nur gleiche Zelltypen in einem Cluster zusammenfinden (nicht gezeigt).

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 1: Vorrichtung
- 2: Kontrolleinheit
- 3: Aufnahmeeinheit
- 4: Petrischale
- 5: Schallgebungseinheit
- 6: Vergrößerungsoptik

## Patentansprüche

1. Vorrichtung (1) zur Identifikation, Separation und/oder zelltypspezifischen Manipulation wenigstens einer Zelle eines Zellsystems sowie von Mikroorganismen, wobei die Vorrichtung (1) eine Schallgebungseinheit (5), eine Kontrolleinheit (2) und eine Aufnahmeeinheit (3) aufweist, wobei die Schallgebungseinheit (5) ein piezo-elektrisches Bauteil aufweist, welches mit einer Frequenz von mehr als 5 MHz gesteuert wird und eine schmale Bandbreite hat.

2. Vorrichtung nach Anspruch 1, **dadurch gekennzeichnet, dass** das piezo-elektrische Bauteil ein piezo-elektrischer Kristall, eine Piezo-Keramik oder ein Piezo-Polymer ist.

3. Vorrichtung nach einem der Ansprüche 1 oder 2, **dadurch gekennzeichnet, dass** das piezo-elektrische Bauteil eine Resonanz von 7 MHz pro Millimeter aufweist.

4. Vorrichtung (1) nach einem der Ansprüche 1 bis 3, **dadurch gekennzeichnet, dass** das piezo-elektrische Bauteil ein Wafer ist, der in einer Orientierung von 36° zur Y-Achse aus einem Lithiumniobatkristall (LiNbO₃) geschnitten ist.

5. Vorrichtung (1) nach einem der Ansprüche 1 bis 4, **dadurch gekennzeichnet, dass** die Kontrolleinheit (2) eine Vergrößerungsoptik (6), bevorzugt ein Binokular oder ein Inversmikroskop ist.

6. Vorrichtung (1) nach einem der Ansprüche 1 bis 5, **dadurch gekennzeichnet, dass** die Aufnahmeeinheit (3) eine Halterung für eine Zellkulturschale, Petrischale (4) o.dgl. ist.

7. Verfahren zur Identifikation, Separation und/oder zelltypspezifischen Manipulation wenigstens einer Zelle eines Zellsystems sowie von Mikroorganismen mit einer Vorrichtung (1) nach wenigstens einem der Ansprüche 1 bis 6, umfassend die Schritte
a) Einbringen des Zellsystems in die Aufnahmeeinheit (3) der Vorrichtung (1),
b) Beschallen des Zellsystems mit der Schallgebungseinheit (5) mit einer zelltypspezifischen Frequenz.

8. Verfahren nach Anspruch 7, **dadurch gekennzeichnet, dass** es wenigstens einen der Schritte
■ Unterscheidung und Sortierung der Zellen des Multizellsystems oder des Zellsystems in primären Zellkulturen;
■ Erstellung eines Resonanzprofils für die beschallten Zellen des Multizellsystems oder des Zellsystems;
■ Transformation der beschallten Zellen des Multizellsystems oder des Zellsystems;
■ Transfektion der beschallten Zellen des Multizellsystems oder des Zellsystems;
■ Diskriminierung der Zellen des Multizellsystems oder des Zellsystems anhand ihres Genotyps, wobei die Zellen bevorzugt Spermien sind;
■ Isolation und Zerstörung von Zellen des Multizellsystems oder des Zellsystems, wobei die Zellen bevorzugt Virus- oder Parasiten-tragende Zellen oder Tumorzellen sind;
■ Einwirken auf die Zellen des Multizellsystems oder des Zellsystems zur Vermeidung von Restenosen, wobei die Zellen bevorzugt Zellen der Neointima sind;
■ Isolation bzw. Identifikation von verschiedenen Subpopulationen innerhalb des Multizellsystems oder des Multizellsystems, wobei das Multizellsystem oder das Zellsystem bevorzugt Mikroorganismen in gemischten Populationen aufweist;
■ Aktivierung von spezifischen dendritischen Zellen;
■ Manipulation von intrazellulärem Signalling umfasst.

9. Verwendung einer Vorrichtung (1) nach wenigstens einem der Ansprüche 1 bis 6 zur Durchführung eines Verfahrens nach einem der Ansprüche 7 bis 8.

10. Verwendung nach Anspruch 9, **dadurch gekennzeichnet, dass** das Verfahren wenigstens einen der Schritte
■ Unterscheidung und Sortierung der Zellen des Multizellsystems oder des Zellsystems in primären Zellkulturen;
■ Erstellung eines Resonanzprofils für die beschallten Zellen des Multizellsystems oder des Zellsystems;
■ Transformation der beschallten Zellen des Multizellsystems oder des Zellsystems;
■ Transfektion der beschallten Zellen des Multizellsystems oder des Zellsystems;
■ Diskriminierung der Zellen des Multizellsystems oder des Zellsystems anhand ihres Genotyps, wobei die Zellen bevorzugt Spermien sind;
■ Isolation und Zerstörung von Zellen des Multizellsystems oder des Zellsystems, wobei die Zellen bevorzugt Virus- oder Parasiten-tragende Zellen oder Tumorzellen sind;
■ Einwirken auf die Zellen des Multizellsystems oder des Zellsystems zur Vermeidung von Restenosen, wobei die Zellen bevorzugt Zellen der Neointima sind;
■ Isolation bzw. Identifikation von verschiedenen Subpopulationen innerhalb des Multizellsystems oder des Zellsystems, wobei das Multizellsystem oder das Zellsystem bevorzugt Mikroorganismen in gemischten Populationen aufweist;
■ Aktivierung von spezifischen dendritischen Zellen;
■ Manipulation von intrazellulärem Signalling umfasst.
